# EUROPÄISCHE PATENTSCHRIFT

(11) **EP 3 125 829 B1**
(45) Veröffentlichungstag und Bekanntmachung des Hinweises auf die Patenterteilung: **08.05.2019**
(21) Anmeldenummer: 15713162.4
(22) Anmeldetag: 25.03.2015
(51) Int. Cl.: A61F 2/30, A61F 2/36

(54) **IMPLANTATVERBINDUNG**
IMPLANT CONNECTION
LIAISON D'IMPLANT

(30) Priorität: 01.04.2014 DE 102014206151
(43) Veröffentlichungstag der Anmeldung: 08.02.2017
(73) Patentinhaber: Waldemar Link GmbH & Co. KG, 22339 Hamburg (DE)
(72) Erfinder: LINK, Helmut D., 22397 Hamburg (DE)
(74) Vertreter: Hoffmann Eitle
(86) Internationale Anmeldenummer: PCT/EP2015/056391
(87) Internationale Veröffentlichungsnummer: WO 2015/150186

(56) Entgegenhaltungen:
- EP-A1- 0 017 743
- EP-A2- 0 864 303
- DE-A1-102009 027 255
- DE-U1-202004 012 349
- DE-U1-202012 104 253
- FR-A1- 2 886 550

## Beschreibung

### TECHNISCHES GEBIET

Die vorliegende Erfindung betrifft eine Implantatkomponente mit einem Verbindungsbereich, wie er bei modularisierten Implantaten eingesetzt wird, und solch ein modularisiertes Implantat.

### STAND DER TECHNIK

Heutige Endoprothesen weisen häufig einen modularen Aufbau auf, d. h. sie sind aus mindestens zwei getrennt voneinander wählbaren Teilen zusammengesetzt. Ein solcher Aufbau verleiht diesen Prothesen eine hohe Anpassungsfähigkeit an die individuellen Anforderungen an die Behandlung eines Patienten.

Die DE 20 2004 012 349 U1 offenbart eine modulare Schenkelhalsprothese für ein künstliches Hüftgelenk mit einem Adapter zur Aufnahme einer künstlichen Gelenkkugel und ist die Grundlage für die zweiteilige Form des ersten Anspruchs.

Die DE 20 2012 104 253 U1 offenbart eine Implantatkomponente mit einem Verbindungsabschnitt, der eine Tantal-Beschichtung mit einer Dicke von 3-50 Mikrometern aufweist.

Der Vorteil des modularen Aufbaus ist, dass trotz der vielfältigen Anpassungsfähigkeit der Endoprothese an die Anatomie und biomechanischen Gegebenheiten eines Patienten nur eine relativ geringe Anzahl von unterschiedlichen Modulen bereitgehalten werden muss. Diese könne zum Beispiel unterschiedliche Formen und/oder Materialien berücksichtigen. Im Ergebnis hat so ein modularer Aufbau insbesondere den Vorteil, dass keine Implantate für jede mögliche Permutation vorrätig gehalten oder individuell hergestellt werden müssen.

Eine modular aufgebaute Endoprothese wird über Verbindungsabschnitte zusammengesetzt. Hierbei haben sich vor allem Konusverbindungen bewährt. Bei ihnen weist eine Implantatkomponente eine weibliche, die andere einen männlichen Konus auf. Beim Zusammenstecken dieser Verbindung wird die durch den Konuswinkel entstehende Hebelwirkung ausgenutzt, um eine Normalkraft auf die Konusflächen aufzubringen, sodass zwischen den beiden Konussen eine reibschlüssige Verbindung erreicht wird. Folglich werden derart ausgeführte Implantatkomponenten miteinander verklemmt.

Beispielsweise finden sich Konusverbindungen zur Verbindung einzelner Implantatkomponenten bei Hüftendoprothesen. Hierbei weisen einfach modulare Hüftendoprothesen eine Implantatverbindung zwischen dem Prothesenschaft und dem Hüftkopf auf.

Nach Einführung derartiger modularer Endoprothesen wurde allerdings das Auftreten von Spaltkorrosion und Reibkorrosion in der Konusverbindung beobachtet, die schlimmstenfalls zu einem fatalen Versagen des Implantats führen kann. Als Ursache für die Korrosion wird von Mikrobewegungen zwischen den Verbindungsflächen zweier Implantatkomponenten und zwischen ihnen auftretenden Spannungen ausgegangen. Die Folge sind Risse und eine Abnutzung der schützenden Oxidschicht auf der Oberfläche des Implantatmaterials, was zu den genannten Korrosionserscheinungen führt.

Diesem wurde durch Änderungen des Prothesendesigns und präziseren Herstellungsverfahren begegnet. Neben der Formgenauigkeit wurde die Oberflächenbeschaffenheit der Kontaktflächen verbessert.

Die nachfolgend auf den Markt gebrachten mehrfach modularen Endoprothesen weisen zusätzlich ein Zwischenstück auf, dass zwischen dem Prothesenschaft und dem Hüftkopf angeordnet ist. Folglich besteht eine Implantatverbindung zwischen dem Prothesenschaft und dem Zwischenstück sowie zwischen dem Zwischenstück und dem Hüftkopf. Prothesensysteme, die ein solches Zwischenstück aufweisen, wurden entwickelt, um eine bessere Anpassung der Version/Anteversion, Länge und Versatzes der Prothese auf die Bedürfnisse des Patienten zu ermöglichen.

Bei diesen mehrfach modularen Prothesen kommt es vorwiegend an der Schnittstelle zwischen dem Prothesenschaft und dem Zwischenstück zu Korrosionserscheinungen. Weiterhin wurde bei modularen Prothesen festgestellt, dass Korrosionserscheinungen nicht nur bei Metall-Metall-Paarungen auftreten, sondern auch bei Metall-Keramik-Paarungen, wie zum Beispiel bei Hüftendoprothesen mit Metallschaft und Keramikkopf.

Als Folge der Korrosion wird zudem das Freisetzen von Metallionen, Metalloxiden, Metallorganophosphaten und kleiner Metallpartikel, die wiederum zu verstärkten mechanischen Abriebserscheinungen führen, beobachtet. Als Folge können Schmerzen, eine aseptische Lockerung und negative Folgewirkungen für das umliegende Gewebe auftreten. Auch kann es zu allergischen Reaktionen kommen, die ebenfalls eine Revision der Prothese erforderlich machen können.

In Anbetracht dessen war es Aufgabe der Erfindung, eine Implantatkomponente bereit zu stellen, die eine Verbindung mit einer weiteren Implantatkomponente zulässt, dabei aber den bekannten obigen Nachteilen für den Patienten bei modularen Prothesen entgegenwirkt.

### ZUSAMMENFASSUNG DER ERFINDUNG UND BEVORZUGTE AUSFÜHRUNGSFORMEN

Um die obige Aufgabe zu lösen, wird durch die vorliegende Erfindung eine Implantatkomponente gemäß Anspruch 1 sowie eine modulare Endoprothese gemäß Anspruch 8 bereitgestellt.

Die erfindungsgemäße Implantatkomponente weist mindestens einen Verbindungsabschnitt auf, der zumindest teilweise mit einer TiNb-Beschichtung beschichtet ist.

Überraschenderweise beugt der erfindungsgemäß beschichtete Verbindungsabschnitt nicht nur allergischen Folgereaktionen vor, sondern gewährt zugleich bei entsprechend gewählter Dicke einen zuverlässigen Reibschluss und führt zu einem besseren Schutz vor Korrosion.

Die Titan-Niob-Beschichtung weist, ähnlich wie Titan, ein hohes Maß an Biokompatibilität auf. Allerdings sind die mechanischen Eigenschaften dieser Metalllegierung besser für die vorliegend vorgesehene Beschichtung geeignet. Insbesondere besitzt die Beschichtung einen gewünscht niedrigen Härtegrad, so dass eine Verklemmung zwischen einer harten Kopplungskomponente und dem beschichteten männlichen Konus durch Zusammenwirken der ursprünglichen Oberflächenstruktur der Konusverbindung gewährleistet ist.

Üblicherweise wird versucht, wie oben bereits dargelegt, einen zuverlässigen Reibschluss durch eine entsprechend bearbeitete Oberfläche des Implantatmaterials im Verbindungsabschnitt zu erreichen. Mit anderen Worten kommt es durch die mechanischen Eigenschaften der Beschichtung beim Aufeinandertreffen des Verbindungsabschnitts auf einen komplementären Verbindungsabschnitt einer weiteren Implantatkomponente zu einer Normalkraft, die eine ausreichende Klemmwirkung über die dadurch entstehende Reibkraft zwischen den Flächen der Verbindungsabschnitte hervorruft.

Üblicherweise werden Nitrid-Beschichtungen verwendet, um die Abriebeigenschaften von Implantatkomponenten zu verbessern. Deswegen werden sie beispielsweise für die Laufflächen von Endoprothesen verwendet, wo jedoch andere Abnutzungsbedingungen vorherrschen als im Verbindungsbereich. Dies mag ein Grund dafür sein, dass Nitridbeschichtungen hier zu keinen zufriedenstellenden Ergebnissen geführt haben.

Die Beschichtung auf dem Verbindungsabschnitt weist dabei vorzugsweise eine Dicke von 1 bis 20, bevorzugt 1-6 µm auf, da in diesem Bereich eine stabile, fortlaufende und belastbare Verbindung der Implantatkomponente erreicht werden kann. Die Beschichtung wird bevorzugt über dem Fachmann bekannte PVD-Verfahren oder Ionenimplantationsverfahren aufgetragen.

Die Beschichtung weist bei einer besonders bevorzugten Ausführungsform eine Schichtdicke von 3 bis 6 *µ*m und noch bevorzugter von 3 bis 5 *µ*m auf.

Ziel ist es, die Dicke der Schicht so hoch zu wählen, dass eine den mechanischen Belastungen widerstehende Beschichtung erzielt wird. Gleichzeitig ist anzustreben, die Beschichtung nicht zu stark zu wählen, damit die Strukturen der Konusoberflächen nicht verdeckt/gestört werden und damit einen stabilen Reibschluss zwischen den Flächen des Verbindungsbereichs nicht entgegenstehen.

Bei einer weiteren bevorzugten Ausführungsform ist der Verbindungsabschnitt durch einen weiblichen und/oder männlichen Konus ausgebildet. Dabei ist der Verbindungsabschnitt so ausgeführt, dass er mit einem komplementär ausgebildeten Konus einer anderen, weiteren Implantatkomponente verbindbar ist.

Bei der anderen Implantatkomponente kann es sich ebenfalls um eine erfindungsgemäße Implantatkomponente mit einem beschichteten Verbindungsabschnitt handeln aber auch um eine beliebige andere Implantatkomponente aus dem Stand der Technik. In beiden Fällen wird der Effekt, einer Korrosion des Verbindungsabschnitts vorzubeugen, grundsätzlich unabhängig von der anderen Implantatkomponente erreicht.

Bei einer besonders bevorzugten Ausführungsform der Implantatkomponente ist der Verbindungsabschnitt rotationssymmetrisch ausgeführt.

Dies ermöglicht eine Ausrichtung der Implantatkomponente um die Rotationsachse des Verbindungsabschnitts und erlaubt somit eine bessere Anpassung an die patientenspezifische Anatomie und biomechanischen Anforderungen.

Es kann aber auch der Fall sein, dass eine Relativbewegung der Implantatkomponente um deren Längsachse gerade nicht gewünscht ist. In solch einem Fall weist der Verbindungsabschnitt keinen rotationssymmetrischen Verbindungsabschnitt auf, um eine Verdrehung der erfindungsgemäßen Implantatkomponente relativ zu der mit ihr zu verbindenden Komponente zu verhindern.

Bei einer bevorzugten Ausführungsform wird ein erfindungsgemäßer Verbindungsabschnitt bei einer Implantatkomponente angewandt, die eine Metalllegierung aufweist.

Dies hat insbesondere Vorteile bei Metalllegierungen, bei denen abgetrennte bzw. abgelöste Bestandteile bekanntermaßen zu negativen Folgewirkungen bei dem betroffenen Patienten führen.

Dementsprechend weist die Implantatkomponente bei einer besonders bevorzugten Ausführungsform eine Kobalt-Chrom-Legierung auf.

Der erfindungsgemäße Verbindungsabschnitt wird bevorzugt bei Gelenkendoprothesen eingesetzt. Dabei kann es sich um den Prothesenschaft, ein Zwischenstück, einen Gelenkkopf und/oder einen sonstigen Bestandteil solch einer Gelenkendoprothese handeln.

Weiterhin wird durch die vorliegende Erfindung eine modulare Endoprothese bereitgestellt, die mindestens eine erfindungsgemäße Implantatkomponente mit einem beschichteten Verbindungsabschnitt nach einer der vorgenannten Ausführungsformen aufweist.

Eine solche modulare Endoprothese weist die anfangs genannten Vorteile eines modularen Aufbaus auf, vermeidet dabei jedoch Korrosionsschäden und deren negative Folgen.

Die Erfindung stellt zudem die Verwendung einer TiNb-Beschichtung zur Vorbeugung gegen Korrosion an einem Verbindungsbereich einer Implantatkomponente bereit, wobei die Beschichtung eine Dicke von 1-20, bevorzugt 1-6 *µ*m aufweist.

Es versteht sich, dass die Beschichtung nicht nur im Verbindungsbereich aufgebracht sein kann, sondern auch auf die darüber hinaus gehende oder die gesamte Implantatoberfläche. Eine derartige Beschichtung hat den Vorteil, dass sie einfacher und damit kostengünstiger hergestellt werden kann, da zum Beispiel der Aufwand einer Maskierung von Abschnitten des Implantats, die nicht beschichtet werden sollen, entfällt.

## Patentansprüche

1. Implantatkomponente, die mindestens einen Verbindungsabschnitt aufweist, **dadurch gekennzeichnet, dass** der Verbindungsabschnitt zumindest teilweise mit einer TiNb-Beschichtung beschichtet ist und die Beschichtung eine Dicke von 1-20, bevorzugt 1-6 µm aufweist.

2. Implantatkomponente nach Anspruch 1, bei der die Beschichtung eine Dicke von 3-6 µm, bevorzugt von 3-5 µm aufweist.

3. Implantatkomponente nach einem der vorstehenden Ansprüche, bei welcher der Verbindungsabschnitt durch einen weiblichen und/oder männlichen Konus ausgebildet ist.

4. Implantatkomponente nach einem der vorstehenden Ansprüche, bei welcher der Verbindungsabschnitt rotationssymmetrisch ist.

5. Implantatkomponente nach einem der vorstehenden Ansprüche, wobei die Implantatkomponente eine Metalllegierung aufweist.

6. Implantatkomponente nach einem der vorstehenden Ansprüche, wobei die Implantatkomponente eine CoCr-Legierung aufweist.

7. Implantatkomponente nach einem der vorstehenden Ansprüche, wobei die Implantatkomponente ein Prothesenschaft, ein Zwischenstück oder ein Gelenkkopf ist.

8. Modulare Endoprothese mit mindestens einer Implantatkomponente nach einem der vorstehenden Ansprüche.

9. Verwendung einer TiNb-Beschichtung zur Vorbeugung gegen Korrosion an einem Verbindungsbereich einer Implantatkomponente, wobei die Beschichtung eine Dicke von 1-20, bevorzugt 1-6 µm aufweist.

## Claims

1. Implant component having at least one connecting portion, **characterized in that** the connecting portion is coated at least partially with a TiNb coating, and the coating has a thickness of 1-20 µm, preferably 1-6 µm.

2. Implant component according to Claim 1, in which the coating has a thickness of 3-6 µm, preferably 3-5 µm.

3. Implant component according to either of the preceding claims, in which the connecting portion is formed by a female and/or male cone.

4. Implant component according to one of the preceding claims, in which the connecting portion is rotationally symmetrical.

5. Implant component according to one of the preceding claims, wherein the implant component has a metal alloy.

6. Implant component according to one of the preceding claims, wherein the implant component has a CoCr alloy.

7. Implant component according to one of the preceding claims, wherein the implant component is a prosthesis shaft, an adapter piece or a joint head.

8. Modular endoprosthesis having at least one implant component according to one of the preceding claims.

9. Use of a TiNb coating to prevent corrosion at a connecting region of an implant component, wherein the coating has a thickness of 1-20 µm, preferably 1-6 µm.

## Revendications

1. Composant d'implant présentant au moins une portion de liaison, **caractérisé en ce que** la portion de liaison est au moins en partie revêtue d'un revêtement de TiNb et le revêtement présente une épaisseur de 1-20, de préférence 1-6 µm.

2. Composant d'implant selon la revendication 1, dans lequel le revêtement présente une épaisseur de 3-6 µm, de préférence de 3-5 µm.

3. Composant d'implant selon l'une quelconque des revendications précédentes, dans lequel la portion de liaison est réalisée par un cône femelle et/ou mâle.

4. Composant d'implant selon l'une quelconque des revendications précédentes, dans lequel la portion de liaison présente une symétrie de révolution.

5. Composant d'implant selon l'une quelconque des revendications précédentes, le composant d'implant présentant un alliage métallique.

6. Composant d'implant selon l'une quelconque des revendications précédentes, le composant d'implant présentant un alliage de CoCr.

7. Composant d'implant selon l'une quelconque des revendications précédentes, le composant d'implant étant une tige de prothèses, une pièce intermédiaire ou une tête d'articulation.

8. Endoprothèse modulaire comprenant au moins un composant d'implant selon l'une quelconque des revendications précédentes.

9. Utilisation d'un revêtement de TiNb pour prévenir contre la corrosion d'une région de liaison d'un composant d'implant, le revêtement présentant une épaisseur de 1-20, de préférence de 1-6 µm.
